# EUROPEAN PATENT APPLICATION

(11) **EP 4 799 612 A1**
(43) Date of publication of application: **02.09.2026**
(21) Application number: 24908111.8
(22) Date of filing: 19.12.2024
(51) Int. Cl.: A61K 9/16, A61K 9/00, A61K 38/26, A61P 3/10

(54) **SUSTAINED-RELEASE INJECTION COMPOSITION COMPRISING EXENATIDE AND METHOD FOR PREPARING SAME**

(30) Priority: 20.12.2023 KR 20230186829
(71) Applicant: Inventage Lab Inc., Gyeonggi-do 13403 (KR)
(72) Inventor: KIM, Ju Hee, Seongnam-si, Gyeonggi-do 13494 (KR); KIM, Min Sung, Seongnam-si, Gyeonggi-do 13494 (KR)
(74) Representative: BCKIP Part mbB
(86) International application number: PCT/KR2024/020702
(87) International publication number: WO 2025/135828

(57) **Abstract**

The present invention relates to a sustained-release injection composition comprising exenatide and a method for preparing same. The sustained-release injection composition enables exenatide to be continuously released in the body for 1-2 months with a single injection, thus making it possible to reduce the number of administrations and improve the convenience of administration. In addition, since the sustained-release injection composition contains exenatide in microparticles having a uniform particle size, long-term sustained release of exenatide can be achieved, and thus the bio-absorption rate of exenatide can be increased compared to conventional injectable formulations comprising exenatide.

## Description

### Technical Field

The present invention relates to a sustained-release injectable composition containing exenatide and a method for preparing the same.

### Background Art

Exenatide is a synthetic peptide of exendin-4, a GLP-1 (glucagon-like peptide 1) analog, and is a drug used to lower blood glucose levels by promoting insulin secretion in patients with type 2 diabetes.

Exenatide has about 53% homology to natural GLP-1, is an agonist for GLP-1 receptors, is resistant to the protease DPP-IV, and has a half-life of 2 to 4 hours. It is injected subcutaneously twice daily at an initial dose of 5 mcg, and may be administered twice daily at a dose of up to 10 mcg. However, its use in type 1 diabetes is not yet approved.

Although several studies on exenatide have consistently shown that exenatide is effective in reducing fasting and postprandial blood glucose levels, glycated hemoglobin levels, and body weight, exenatide has a short *in vivo* half-life, and thus efforts have been made to improve the half-life.

Therefore, there is a need to develop high-efficiency formulations containing exenatide to improve patient convenience, such as reducing the frequency of administration, and to reduce manufacturing costs.

### [Prior Art Documents]

### [Patent Documents]

KR 10-2018-0129825 A1

### DISCLOSURE

### Technical Problem

An object of the present invention is to provide a sustained-release injectable composition containing exenatide and a method for preparing the same.

Another object of the present invention is to provide a sustained-release injectable composition that is capable of releasing exenatide *in vivo* in a sustained manner for 1 to 2 months by a single injection, thereby reducing the number of administrations and increasing the convenience of administration.

Still another object of the present invention is to provide a method for preparing a sustained-release injectable composition containing exenatide, which contains exenatide within microparticles with a uniform particle size, and thus is capable of exhibiting the effect of releasing exenatide in a sustained manner over a long period of time, thereby increasing the bioavailability of exenatide compared to conventional injectable formulations of exenatide.

### Technical Solution

To achieve the above objects, the present invention relates to a sustained-release injectable composition containing exenatide, which comprises microparticles, wherein the microparticles comprise exenatide and a biodegradable polymer, and the area under the plasma level-time curve (AUC) for the release of exenatide for 63 days after administering the sustained-release injectable composition to SD rats at an exenatide dose of 8 mg/kg is 40,000 pg·d/mL to 100,000 pg·d/mL.

In addition, as the sustained-release injectable composition is administered to SD rats at an exenatide dose of 8 mg/kg, it may exhibit an effect of releasing exenatide rapidly at an initial stage, and subsequently maintain a state in which the plasma concentration of exenatide is steady.

In addition, the above sustained-release injectable composition is capable of releasing exenatide in a sustained manner for 1 to 2 months.

In addition, the microparticles may comprise exenatide and the biodegradable polymer at a weight ratio of 1:3 to 1:7.

In addition, the microparticles may have an average diameter of 40 µm to 80 µm.

In addition, the biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

In addition, the injectable composition may comprise a suspending solvent.

A method for preparing a sustained-release injectable composition containing exenatide according to another embodiment of the present invention comprises: step 1) of preparing an oil phase solution by mixing exenatide and a biodegradable polymer with an organic solvent; step 2) of preparing a water phase solution by dissolving a surfactant in purified water; step 3) of injecting the oil phase solution and the water phase solution into microchannels, respectively, and allowing the oil phase solution and the water phase solution to flow, thereby producing microparticles at an intersection formed between the microchannels; step 4) of collecting the microparticles in a bath containing the water phase solution; step 5) of removing the organic solvent from the collected microparticles; step 6) of washing the microparticles, from which the organic solvent has been removed, with purified water, followed by drying; and step 7) of mixing the dried microparticles with a suspending solvent, thereby preparing the sustained-release injectable composition, wherein the area under the plasma level-time curve (AUC) for the release of exenatide for 63 days after administering the sustained-release injectable composition to SD rats at an exenatide dose of 8 mg/kg may be 40,000 pg·d/mL to 100,000 pg·d/mL.

In addition, the oil phase solution may be prepared by dissolving exenatide in a co-solvent and then mixing the solution with the biodegradable polymer and the organic solvent.

The co-solvent may be selected from the group consisting of acetic acid, dimethyl sulfoxide, dimethylformamide, tetrahydrofuran, ethyl acetate, acetonitrile, and mixtures thereof.

In addition, the co-solvent may be contained at a weight ratio of 1:1 to 1:2 relative to the organic solvent.

In addition, when the oil phase solution is injected into the microchannel, it may be injected under a pressure condition of 500 mbar to 700 mbar, and then the pressure may be increased at a rate of 1 to 5 mbar/min.

In addition, the water phase solution may be injected into the microchannel under a pressure condition of 2,500 mbar to 3,000 mbar.

In addition, step 5) may comprise stirring at a speed of 200 to 400 rpm for 4 to 6 hours at 40 to 50°C.

### Advantageous Effects

The sustained-release injectable composition according to the present invention is capable of releasing exenatide *in vivo* in a sustained manner for 1 to 2 months by a single injection, thereby reducing the number of administrations and increasing the convenience of administration.

In addition, the sustained-release injectable composition contains exenatide within microparticles with a uniform particle size, and thus is capable of exhibiting the effect of releasing exenatide in a sustained manner over a long period of time, thereby increasing the bioavailability of exenatide compared to conventional injectable formulations of exenatide.

### Brief Description of Drawings

FIG. 1 shows SEM photographs of microparticles according to one embodiment of the present invention.
FIG. 2 shows SEM photographs of microparticles according to one embodiment of the present invention.
FIG. 3 shows SEM photographs of microparticles according to one embodiment of the present invention.
FIG. 4 shows SEM photographs of microparticles according to one embodiment of the present invention.
FIG. 5 shows PK measurement results for an injectable composition according to one embodiment of the present invention.
FIG. 6 shows PK measurement results for an injectable composition according to one embodiment of the present invention.

### Best Mode

The present invention relates to a sustained-release injectable composition containing exenatide, which comprises microparticles, wherein the microparticles comprise exenatide and a biodegradable polymer, and the area under the plasma level-time curve (AUC) for the release of exenatide for 63 days after administering the sustained-release injectable composition to SD rats at an exenatide dose of 8 mg/kg is 40,000 pg·d/mL to 100,000 pg·d/mL.

### Mode for Invention

Hereinafter, embodiments of the present invention will be described in detail so that those skilled in the art can easily implement the present invention. However, the present invention may be embodied in various different forms and is not limited to the embodiments described herein.

Exenatide is a 39-amino acid peptide that is a potent agonist of the GLP-1 receptor, making it an insulin secretagogue with glucoregulatory effects. It is a free peptide marketed as Byetta^{®} (Astra-Zeneca) and is widely used in the treatment of type 2 diabetes. Because this peptide has a short *in vivo* half-life of 2.5 hours, it is injected twice a day.

It is highly desired to extend the half-life of exenatide and related GLP-1 agonist peptides in order to improve the efficacy thereof, reduce the side effects thereof, and reduce the burden of treatment on patients.

Peptide half-life is traditionally extended by one or a combination of several methods: (i) chemical modification of the peptide to slow metabolism; (ii) encapsulation to provide a slow-release depot formulation; and (iii) conjugation with a macromolecule to slow *in vivo* clearance [see, for example, Cal, et al., Drug Design, Development, and Therapy (2013) 7:963-970].

Chemical modifications of the peptide to increase half-life have resulted in once-daily GLP-1 agonists, for example, lixisenatide (Lyxumia^{®}) and liraglutide (Victoza^{®}). Encapsulation of the peptide into PLGA (poly lactic-coglycolic acid) microparticles has been used to produce a slow-release formulation, marketed as Bydureon^{®} (Astra-Zeneca), that allows for once-weekly subcutaneous injection.

Although Bydureon^{®} is released for 10 weeks even with a single administration, there is a problem in that the patient is not exposed to exenatide to a sufficient degree to exhibit a therapeutic effect on type 2 diabetes through the release of exenatide. Therefore, Bydureon^{®} is used to treat type 2 diabetes with exenatide by once-weekly subcutaneous injection.

However, in the case of once-weekly subcutaneous injection as described above, the inconvenience of having to administer a subcutaneous injection every week arises. Thus, there is a need to develop a formulation that can exhibit the effect of releasing exenatide for a longer period of time by a single administration.

The present invention is intended to resolve the above-described problem and relates to a sustained-release injectable composition comprising microparticles that can release exenatide for 1 to 2 months by a single injection as described below, thereby improving the convenience of administration.

Specifically, the microparticles may comprise exenatide and a biodegradable polymer, and the area under the plasma level-time curve (AUC) for the release of exenatide for 63 days after administering the sustained-release injectable composition to SD rats at an exenatide dose of 8 mg/kg may be 40,000 pg·d/mL to 100,000 pg·d/mL.

The AUC is related to the bioavailability following release of exenatide release, and a higher AUC value indicates higher bioavailability.

The sustained-release injectable composition of the present invention may have an area under the plasma level-time curve (AUC) of 40,000 pg·d/mL to 100,000 pg·d/mL, 45,000 pg·d/mL to 60,000 pg·d/mL, 50,000 pg·d/mL to 60,000 pg·d/mL, or 55,000 pg·d/mL to 580,000 pg·d/mL for the release of exenatide for 63 days after administering exenatide to SD rats at a dose of 8 mg/kg. The AUC value of the sustained-release injectable composition of the present invention shows a significant difference from that of the above-described Bydureon^{®}.

That is, even when treated with the same concentration of exenatide as Bydureon^{®} that is administered once a week, the sustained-release injectable composition containing exenatide of the present invention shows a large difference in AUC value, and thus may exhibit excellent bioavailability.

The above-described AUC value is due to the characteristics of the microparticles included in the sustained-release injectable composition. The microparticles of the present invention are spherical in shape with a smooth surface, and have an average diameter of 40 µm to 80 µm, 60 µm to 80 µm, or 70 µm to 80 µm, and the standard deviation (SD) for the average diameter of the particles is 7 to 8, indicating that the particles have a very uniform average diameter.

On the other hand, the average diameter of the microparticles of Bydureon^{®} is 76.51 µm, which is not significantly different from that of the microparticles of the present invention, but the surface of the particles is not uniform, so there is a significant difference in shape. In addition, the standard deviation (SD) for the average diameter of the particles is 24.99, indicating that the particles do not have a uniform diameter. Thus, the microparticles of Bydureon^{®} are significantly different from the microparticles of the present invention.

Due to the above-described differences in particle characteristics, the sustained-release injectable composition of the present invention can exhibit the effect of releasing exenatide in a sustained manner for 1 to 2 months by a single administration.

In addition, the sustained-release injectable composition is administered to SD rats at an exenatide dose of 8 mg/kg, it may exhibit an effect of releasing exenatide rapidly at an initial stage, and subsequently maintain a steady state. That is, the sustained-release injectable composition of the present invention is characterized in that, when it is administered to SD rats, the initial rapid release of exenatide appears, and then the plasma concentration of exenatide may be continuously maintained at a certain level or higher.

Conventional microparticles having a uniform diameter are characterized by releasing the drug contained in the microparticles in a sustained manner without initial rapid drug release, but the microparticles included in the sustained-release injectable composition of the present invention differ in that they exhibit the rapid release of exenatide immediately after injection, and then exhibit a pattern of releasing exenatide at a certain level or higher in a sustained manner.

When the sustained-release injectable composition of the present invention is administered to SD rats in the manner described above and the pattern of exenatide release in the blood is checked, it can be confirmed that after the initial rapid release of exenatide, the plasma concentration of exenatide is maintained for a long time. In contrast, it could be confirmed that, when Bydureon^{®} was administered to SD rats at the same dose, it exhibited a sustained release pattern without the initial rapid release of exenatide, unlike the release pattern of the present invention, but from the time of the first injection until day 21, the plasma concentration of exenatide was excessively low, making it difficult to exert the effect of exenatide, and after day 42, the plasma concentration decreased rapidly, indicating that Bydureon^{®} cannot exhibit the same effect as that of the present invention by a single administration.

The microparticles are characterized by comprising exenatide and the biodegradable polymer at a weight ratio of 1:3 to 1:7, preferably 1:5, without being limited to the above example. When the microparticles comprise exenatide and the biodegradable polymer at a weight ratio within the above range, they can exhibit an excellent effect of releasing exenatide in a sustained manner.

The biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof, and preferably, may be selected from the group consisting of polylactic acid, polylactide-co-glycolide (PLGA), and a mixture thereof, but the biodegradable polymer is not limited to the above examples, and any biodegradable polymer may be used without limitation as long as it may be degraded *in vivo* and release exenatide in a sustained manner.

Specifically, the biodegradable polymer may be PLGA, and may comprise a mixture of two types of PLGA, that is, PLGA with a viscosity of 0.16 to 0.24 dl/g and PLGA with a viscosity of 0.35 to 0.45 dl/g. When the biodegradable polymer comprises two types of PLGA with different viscosities, it may exhibit the exenatide release pattern unique to the present invention, and thus may exhibit the sustained release of exenatide for 1 to 2 months and exhibit the effects resulting from the release of exenatide.

The injectable composition may comprise a suspending solvent. The suspending solvent comprises an isotonic agent, a suspending agent, and a solvent.

More specifically, the isotonic agent may be selected from the group consisting of D-mannitol, maltitol, sorbitol, lactitol, xylitol, sodium chloride, and mixtures thereof, preferably D-mannitol, but the isotonic agent is not limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, and is preferably sodium carboxymethylcellulose and polysorbate 80, but the suspending agent is not limited to the above examples.

As the solvent, water for injection may be used, and any solvent that may be used as water for injection may be used without limitation.

A method for preparing a sustained-release injectable composition containing exenatide according to another embodiment of the present invention comprises: step 1) of preparing an oil phase solution by mixing exenatide and a biodegradable polymer with an organic solvent; step 2) of preparing a water phase solution by dissolving a surfactant in purified water; step 3) of injecting the oil phase solution and the water phase solution into microchannels, respectively, and allowing the oil phase solution and the water phase solution to flow, thereby producing microparticles at an intersection formed between the microchannels; step 4) of collecting the microparticles in a bath containing the water phase solution; step 5) of removing the organic solvent from the collected microparticles; step 6) of washing the microparticles, from which the organic solvent has been removed, with purified water, followed by drying; and step 7) of mixing the dried microparticles with a suspending solvent, thereby preparing the sustained-release injectable composition, wherein the area under the plasma level-time curve (AUC) for the release of exenatide for 63 days after administering the sustained-release injectable composition to SD rats at an exenatide dose of 8 mg/kg may be 40,000 pg·d/mL to 100,000 pg·d/mL.

Step 1) is a step of preparing an oil phase solution, which is a step of preparing an oil phase solution by dissolving exenatide and a biodegradable polymer in an organic solvent.

However, the oil phase solution may be prepared by dissolving exenatide in a co-solvent and then mixing the solution with a biodegradable polymer and an organic solvent.

The co-solvent may be selected from the group consisting of acetic acid, dimethyl sulfoxide, dimethylformamide, tetrahydrofuran ethyl acetate, acetonitrile, and mixtures thereof, and preferably may be acetic acid and dimethyl sulfoxide.

When the co-solvent is used to dissolve exenatide as described above and then the oil phase solution is prepared, the co-solvent may not only help dissolve exenatide, but also induce the rapid initial release of exenatide after the produced microparticles are administered *in vivo*, compared to when the co-solvent is not used. In addition, compared to when one type of co-solvent is used, when acetic acid and dimethyl sulfoxide are used as described above, not only the initial rapid release of exenatide from the microparticles may be induced, but the bioavailability of exenatide *in vivo* may also be increased as described above, and the effect of releasing exenatide in a sustained manner for a long period of time may be exhibited.

When the co-solvent is used to dissolve exenatide, a first co-solvent and a second co-solvent may be used, wherein the first co-solvent is acetic acid and the second co-solvent is dimethyl sulfoxide. The first co-solvent and the second co-solvent may be mixed together at a weight ratio of 1:1 to 1:5, at a weight ratio of 1:2 to 1:4, or at a weight ratio of 1:3. When the first co-solvent and the second co-solvent are mixed at a weight ratio within the above range and used, not only they may facilitate the dissolution of exenatide, but the initial rapid release of exenatide from the produced microparticles may also be induced, the bioavailability of exenatide may be improved, and the effect of releasing exenatide in a sustained manner for a long period of time may be exhibited.

As described above, the oil phase solution may be prepared by dissolving exenatide using the co-solvent and then mixing the solution with the biodegradable polymer and the organic solvent.

At this time, the ratio of the total weight of the co-solvent to the weight of the organic solvent in the oil phase solution may be 1:1 to 1:2. When the co-solvent and the organic solvent are mixed at a weight ratio within the above range and used, exenatide and the biodegradable polymer may be completely dissolved in the organic solvent, so that the oil phase solution may be prepared and microparticles with a uniform surface may be produced by the production process described below.

The biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalate, polyhydroxybutyrate, polyamino acids, and combinations thereof, and is preferably polylactide-co-glycolide (PLGA) and/or polylactide (PLA), but the biodegradable polymer is not limited to the above examples.

In addition, the organic solvent is water-immiscible, and is, for example, any one or more selected from the group consisting of chloroform, chloroethane, dichloroethane, trichloroethane, and mixtures thereof, preferably dichloromethane, but the organic solvent is not limited to the above examples. However, the organic solvent is not limited to the above examples, and any organic solvent capable of dissolving the biodegradable polymer and exenatide may be used as long as it may be easily selected by those skilled in the art.

The weight ratio between exenatide and the biodegradable polymer in the oil phase solution may be 1:3 to 1:7, 1:4 to 1:6, or 1:5. When exenatide and the biodegradable polymer are mixed and used at a weight ratio within the above ranges, exenatide may be released in a sustained manner for a long period of time due to the degradation of the biodegradable polymer.

When the weight ratio between exenatide and the biodegradable polymer is less than 1:3, that is, when the biodegradable polymer is contained in an amount less than the above weight ratio, the ratio of the weight of the biodegradable polymer to that of exenatide is small, and thus a problem may arise in that it is difficult to produce sustained-release particles in which exenatide is uniformly distributed and contained in spherical biodegradable polymer particles. When the weight ratio between the biodegradable polymer and exenatide is more than 1:7, that is, when the biodegradable polymer is contained in an amount more than the above weight ratio, the content of exenatide in the sustained-release particles is low, and thus a problem may arise in that a large amount of sustained-release particles should be administered in order to administer the drug at a desired concentration.

More specifically, the biodegradable polymer in the above oil solution may be contained in an amount of 5 to 15 w%, 6 to 12 wt%, 7 to 10 wt%, or 9.3 wt%, without being limited to the above example.

Step 2) is a step of preparing a water phase solution by dissolving a surfactant in purified water. As the surfactant, any surfactant may be without limitation as long as it may help the biodegradable polymer solution to form a stable emulsion. Specifically, the surfactant is any one or more selected from the group consisting of nonionic surfactants, anionic surfactants, cationic surfactants, and mixtures thereof, and more specifically, is any one or more selected from the group consisting of methylcellulose, polyvinylpyrrolidone, lecithin, gelatin, polyvinyl alcohol, polyoxyethylene sorbitan fatty acid ester, polyoxyethylene castor oil derivatives, sodium lauryl sulfate, sodium stearate, ester amine, linear diamine, fatty amines, and mixtures thereof, preferably polyvinyl alcohol, but the surfactant is not limited to the above examples.

The content of the surfactant in the water phase solution may be 0.1 to 1.0 wt%, 0.3 to 0.7 wt%, or 0.5 wt%. The rest is purified water.

Step 3) is a step of injecting the oil phase solution and the water phase solution into microchannels formed on a wafer and allowing the oil phase solution and the water phase solution to flow.

More specifically, the microchannels may be formed on a material selected from the group consisting of a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited to the above examples.

As an example, aluminum is deposited on a silicon wafer using an e-beam evaporator, and photoresist is patterned on the aluminum using a photolithography technique. Thereafter, the aluminum is etched using the photoresist as a mask, the photoresist is removed, and then the silicon wafer is etched by deep ion reactive etching (DRIE) using the aluminum as a mask. After the aluminum is removed, glass is anodically bonded onto the wafer and hermetically sealed, thereby fabricating the microchannels.

As the microchannel for producing the microparticles of the present invention, a 90-channel chip may be used. In the case of the microchip, the average diameter of the microchannels into which the oil phase solution and the water phase solution are to be injected is 300 µm to 500 µm, and the oil phase solution and the water phase solution may pass through resistance channels after moving through the respective channels. The average diameter of the resistance channels is 10 µm to 50 µm. After passing through the resistance channels, the oil phase solution and the water phase solution pass through a junction channel where they intersect, and the diameter of the junction channel may be 100 µm to 400 µm. After the oil phase solution and the water solution intersect each other within the junction channel to form an emulsion, they pass through a microchannel with a diameter of 150 µm to 200 µm, and then pass through a microchannel with a diameter of 200 µm to 300 µm.

However, the average diameter of the microchannels may vary depending on the range of injection pressure. In addition, the average diameter of the microchannels is closely related not only to the average diameter of the particles, but is also to the injection pressure of the oil phase solution and the water phase solution.

In addition, step 3) is a step of injecting the oil phase solution and the water phase solution into the first microchannel and the second microchannel, respectively, which have an intersection formed therebetween, and allowing the oil phase solution and the water phase solution to flow under the injection pressure conditions.

That is, the oil phase solution flows along the first microchannel, and the water phase solution flows along the second microchannel configured to form an intersection with the first microchannel, and meets the flow of the oil phase solution.

More specifically, the oil phase solution may be injected into the first microchannel under a pressure condition of 600 mbar to 700 mbar, and then the pressure may be increased at a rate of 1 to 5 mbar/min.

In addition, the water phase solution may be injected into the second microchannel under a pressure condition of 2,500 mbar to 3,000 mbar, 2,600 mbar to 2,900 mbar, or 2,800 mbar.

Specifically, in the production method using the microchannels, when the flow rates of the oil phase solution and the water phase solution flowing inside the microchannels were set to certain values using a flow meter and the pressure was measured through feedback control, it was confirmed that the pressure required for the oil phase solution to flow through the microchannel at a certain flow rate increased gradually over time.

Therefore, it is possible to minimize the flow rate variability by using a method of constantly increasing the pressure applied to the oil phase solution, and to prevent the problem of non-uniform microparticle distribution or channel closure due to slow curing of the oil phase solution inside the microchannel, and to increase the production yield of desired microparticles.

In addition, the pressure conditions used when injecting the oil phase solution and the water phase solution into the microchannels serve to control the average diameter of the produced microparticles, and if the above-described range is not specifically satisfied, a problem may arise in that the size of the produced particles is not uniform, or the average diameter range of the microparticles of the present invention is not satisfied.

That is, in order to increase the flow rate of the water phase solution, which forms an intersection with the flow of the oil phase solution, compared to the flow rate of the oil phase solution injected into the microchannel, the water phase solution is allowed to flow under a higher pressure condition.

As described above, when the flow rates of the oil phase solution and the water phase solution are made different from each other and the flow rate of the water phase solution is increased compared to the flow rate of the oil phase solution, the water phase solution having a relatively higher flow rate compresses the oil phase solution at the point where the flow of the oil phase solution and the flow of the water phase solution meet each other, and in this case, due to repulsive force between the oil phase solution and the water phase solution, the biodegradable polymer and exenatide in the oil phase solution form spherical microparticles, and more specifically, form microparticles in which exenatide is uniformly distributed in the spherical biodegradable polymer.

Step 4) is a step of collecting microparticles. In this step, the microparticles are collected in a bath containing the water phase solution to prevent aggregation of initially produced microparticles.

Step 4) is performed using the water phase solution prepared in step 2), that is, a mixed solution of the surfactant and purified water. Specifically, a portion of the water phase solution prepared in step 2) is injected into the microchannel, and the other portion is transferred into the bath in step 4) and used to prevent aggregation of the collected microparticles.

Step 5) is a step of removing an organic solvent from the microparticles collected in the bath. In this step, an organic solvent present on the surfaces of the sustained-release microparticles is evaporated and removed by stirring the microparticles at a predetermined stirring speed at a predetermined temperature. Here, the stirring may be performed at 200 rpm to 400 rpm at 40°C to 50°C for 4 hours to 6 hours. Specifically, the stirring may be performed at 250 rpm to 350 rpm at 40°C to 45°C for 4.5 hours to 5.5 hours.

As the residual organic solvent is removed under the above-described stirring conditions, formation of pores on the surface of the microparticles may be prevented, enabling the production of microparticles with a smooth surface, and the residual organic solvent may also be minimized.

The temperature at which the oil phase solution and the water phase solution flow through the microchannels is also 15 to 20°C, preferably 18°C. That is, after flowing through the microchannels and forming microparticles at the intersection, the temperature is constantly maintained at a low temperature of 15 to 20°C until the collected microparticles are subjected to first stirring. Only when a low temperature is maintained during the microparticle production process, spherical particles may be produced and maintained. In other words, if the low temperature conditions are not met, a problem arises in that it is difficult to produce particles with a consistent spherical shape.

Lastly, step 6) is a step of washing and drying the microparticles. In this step, the microparticles from which the organic solvent on the surface has been completely removed by stirring are washed several times with sterile filtered purified water to remove the surfactant remaining on the microparticles, and then the microparticles are freeze-dried.

The finally produced microparticles are in a form in which exenatide is uniformly distributed in spherical microparticles made of the biodegradable polymer, and comprise exenatide and the biodegradable polymer at a weight ratio of 1:3 to 1:7.

The weight ratio between exenatide and the biodegradable polymer in the microparticles is the same as the weight ratio in the oil phase solution. Specifically, as the organic solvent is completely evaporated and removed from the produced microparticles, the produced microparticles may contain exenatide and the biodegradable polymer at the same weight ratio as the weight ratio in the oil phase solution.

The produced microparticles may be prepared into an injectable composition by mixing with a suspending solvent. The description of the suspending solvent is as described above.

### Production Example 1

### Production of Microparticles Containing Exenatide

An API mixture was prepared by dissolving exenatide in acetic acid and dimethyl sulfoxide. An oil phase solution was prepared by dissolving PDLG7502A, PDLG7504A, and dichloromethane in the API mixture. The weight ratio between exenatide and the biodegradable polymer in the oil phase solution was 1:5, and the biodegradable polymer in the oil phase solution was contained in an amount of 9.3 wt%.

A water phase solution containing 0.5 wt% of polyvinyl alcohol was prepared by mixing polyvinyl alcohol as a surfactant with purified water.

The oil phase solution and the water phase solution were injected into microchannels formed on a silicon wafer and allowed to flow.

Here, in order to allow each of the oil phase solution and the water phase solution to flow at a constant flow rate, the oil phase solution was allowed to flow under a pressure condition starting from 650 mbar, which was increased at a constant rate of 2 mbar/min, and the water phase solution was allowed to flow under a pressure condition of 2,800 mbar. The temperature was maintained at 18°C, and the stirring speed was maintained at 200 rpm.

Microparticles produced at the intersection between the flow of the oil phase solution and the flow of the water phase solution were collected in a bath containing the water phase solution. The microparticles collected in the bath were stirred at a speed of 300 rpm for 5 hours at 43°C.

After completion of stirring, the microparticles were washed several times with sterile filtered purified water and freeze-dried, thereby producing microparticles.

### Production Examples 2 and 3

Microparticles were prepared in the same manner as in Production Example 1, except that the type of biodegradable polymer, the mixing ratio between the two types of biodegradable polymers, the injection pressure of the oil phase solution, and the temperature condition for removing residual solvent were changed.

### Examples 1 to 3

The microparticles of each of Production Examples 1 to 3 above were added to 0.25 ml of a suspending solvent per vial and then uniformly suspended, thereby preparing compositions for subcutaneous injection. The weight ratio between exenatide and the biodegradable polymer in each composition for subcutaneous injection was 1:5.

The suspending solvent had the composition shown in Table 1 below.

**[Table 1]**

| Volume | Purpose of mixing | Component name | Quantity | Unit |
|---|---|---|---|---|
| 0.25 mL | Isotonic agent | D-mannitol | 12.5 | mg |
| | Suspending agent | Sodium carboxymethylcellulose | 0.625 | mg |
| | Suspending agent | Polysorbate 80 | 0.25 | mg |
| | Solvent | Water for injection | Remainder | |

As a comparative example, commercially available Bydureon BCise was purchased and used. The production conditions in Production Examples 1 to 3 above are shown in Table 2 below:

**[Table 2]**

| | Oil phase solution | | | Water phase solution (PVA, %) | Oil phase pressure (mbar) | Solvent removal | | |
|---|---|---|---|---|---|---|---|---|
| | Polymer | Co-solvent | Solvent | | | Temperature (°C) | Stirring (rpm) | Time (h) |
| Production Example 2 | PDLG7504A(3) and PDL02A(1) | Acetic acid and DMSO | DCM | 0.5 | 550 (up3/min) | 43 | 300 | 5 |
| Production Example 3 | PDLG7504A | Acetic acid and DMSO | DCM | 0.5 | 600 (up2/min) | 43.5 | 300 | 5 |
| Production Example 1 | PDLG7502A(1) and PDLG7504A(1) | Acetic acid and DMSO | DCM | 0.5 | 650 (up2/min) | 42.5 | 300 | 5 |

### Experimental Example 1

### Examination of Characteristics of Microparticles

To specifically examine the diameters of the microparticles, analysis was performed using a Microtrac particle size analyzer.

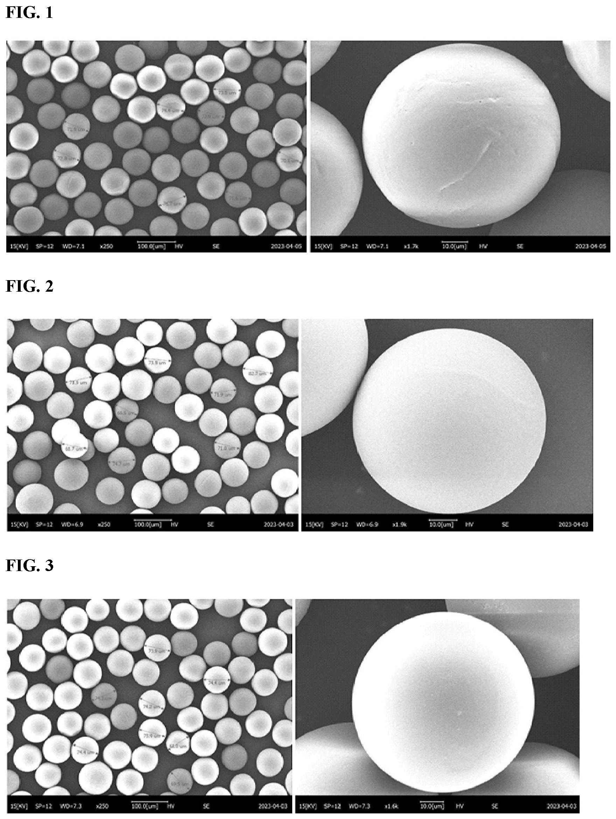

The measurement results are shown in FIGS. 1 to 4 and Table 3 below:

**[Table 3]**

| %Tile | Production Example 1 | Production Example 2 | Production Example 3 | Comparative Example |
|---|---|---|---|---|
| 10.00 | 64.88 | 65.14 | 65.20 | 47.73 |
| 20.00 | 66.95 | 67.38 | 67.49 | 55.61 |
| 30.00 | 68.90 | 69.43 | 69.57 | 62.73 |
| 40.00 | 70.78 | 71.44 | 71.62 | 69.66 |
| 50.00 | 72.78 | 73.48 | 73.70 | 76.51 |
| 60.00 | 74.93 | 75.69 | 75.93 | 83.49 |
| 70.00 | 77.34 | 78.05 | 78.32 | 90.77 |
| 80.00 | 80.21 | 80.63 | 80.92 | 98.82 |
| 90.00 | 83.76 | 83.84 | 84.10 | 109.7 |
| 95.00 | 86.23 | 86.01 | 86.34 | 118.3 |
| SD | 7.71 | 7.71 | 7.81 | 24.99 |

As shown in Table 3 above, it can be confirmed that the microparticles of Production Examples 1 to 3 of the present invention have D50s of 72.78 µm, 73.48 µm, and 73.70 µm, and standard deviations of 7.71, 7.71, and 7.81, indicating that they have a uniform diameter. In contrast, it can be confirmed that the Comparative Example has a D50 of 76.51 µm, which is not significantly different from that of the microparticles of the present invention, but the standard deviation is 24.99, indicating a large difference in diameter between the particles, suggesting that the diameter is not uniform,

### Experimental Example 2

### Evaluation of Pharmacokinetic Properties

Pharmacokinetic evaluations of Examples 1 to 3 of the present invention and the Comparative Examples were performed.

**[Table 4]**

| | Formulation | Dose (mg) | Solvent |
|---|---|---|---|
| Comparative Example | Bydureon Bcise (QW_4) | 2.0 | MCT oil |
| Example 2 | API(1) : (5)[PDLG7504A(3) : PDL02A(1)] | 2.0 | Aqueous solution |
| Example 3 | API(1) : (5)[PDLG7504A] | 2.0 | Aqueous solution |
| Example 1 | API(1) : (5)[PDLG7502A(1) : PDLG7504A(1)] | 2.0 | Aqueous solution |

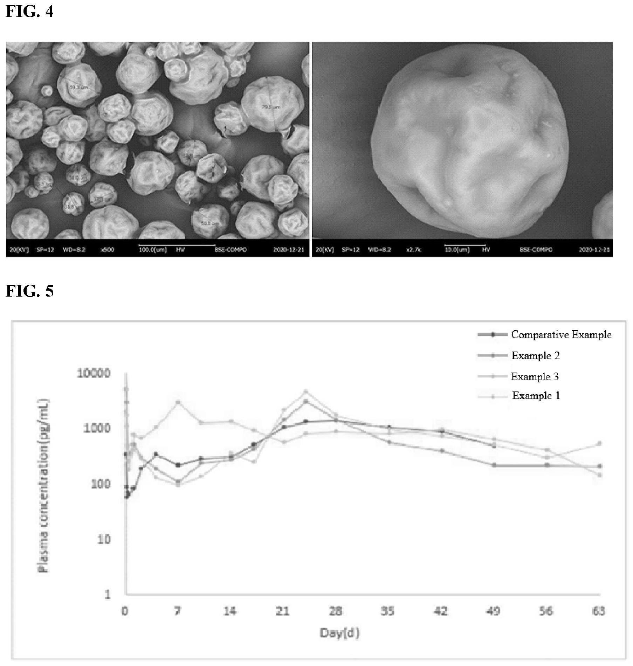

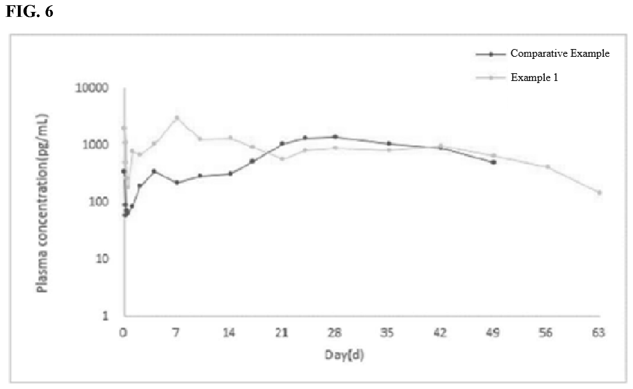

For evaluation, each of Examples 1 to 3 and the Comparative Example was administered to SD rats, blood was collected, and the plasma concentration (PK) of exenatide was measured. The test results are shown in FIGS. 5 and 6 and Table 5 below:

**[Table 5]**

| Dose | 8 mg/kg | 8 mg/kg | 8 mg/kg | 8 mg/kg |
|---|---|---|---|---|
| Day | Exenatide SC | | | |
| d | Example 2 | Example 3 | Example 1 | Comparative Example |
| 0 | ND | ND | ND | ND |
| 0.02 | 5097.825 | 10618.185 | 2035.423 | 346.552 |
| 0.08 | 2985.977 | 5100.142 | 1131.441 | 89.03 |
| 0.17 | 1121.320 | 1770.526 | 501.061 | 57.901 |
| 0.33 | 489.333 | 441.056 | 261.027 | 69.414 |
| 0.5 | 337.369 | 253.732 | 178.898 | 63.716 |
| 1 | 512.932 | 409.931 | 784.918 | 85.946 |
| 2 | 294.111 | 281.292 | 671.312 | 191.19 |
| 4 | 188.902 | 133.943 | 1060.353 | 338.895 |
| 7 | 108.439 | 97.162 | 3052.336 | 217.643 |
| 10 | 235.109 | 137.477 | 1286.705 | 289.200 |
| 14 | 275.403 | 364.087 | 1305.332 | 316.246 |
| 17 | 437.362 | 248.734 | 927.035 | 508.866 |
| 21 | 1482.065 | 2163.971 | 575.203 | 1042.042 |
| 24 | 3188.742 | 4821.117 | 821.839 | 1330.108 |
| 28 | 1460.001 | 1746.177 | 871.766 | 1419.038 |
| 35 | 570.695 | 974.335 | 816.499 | 1054.611 |
| 42 | 389.163 | 743.981 | 958.549 | 879.354 |
| 49 | 218.772 | 514.262 | 645.167 | 483.874 |
| 56 | 213.625 | 297.446 | 417.987 | BQL |
| 63 | 209.278 | 526.694 | 143.874 | ND |

Referring to Table 5 above, it can be confirmed that the commercially available Comparative Example showed a pattern of releasing a small amount of exenatide at the initial stage after injection, and then the release of exenatide continued to increase, but the release of exenatide ended quickly compared to the injectable composition of the present invention. In contrast, it could be confirmed that Example 1 showed rapid release of exenatide immediately after administration, and then exhibited the effect of releasing exenatide in a sustained manner.

On the other hand, it can be confirmed that, in the Comparative Example, the effect of releasing exenatide at the initial stage was insufficient, and thus it is necessary to establish a steady state through repeated administration at weekly intervals. In addition, it can be confirmed that, in the case of Examples 2 and 3, rapid initial release of exenatide appeared, but subsequent release of exenatide was insufficient, and thus the effect of exenatide hardly appeared.

The results of comparing PK data between the Comparative Example and Example 1 to confirm more clearly are shown in FIG. 6. Referring to FIG. 6, it can be confirmed that, when the sustained-release injectable composition of the present invention was administered, rapid initial release of exenatide appeared and sustained release of exenatide appeared, unlike the Comparative Example. On the other hand, it can be confirmed that, in the Comparative Example, the release of exenatide from the initial stage to day 21 after injection was insufficient.

The results of comparing the PK parameters between the Comparative Example and Example 1 are shown in Table 6 below:

**[Table 6]**

| PK Parameters | Exenatide SC | |
|---|---|---|
| | Comparative Example | Example 1 |
| AUC (last) | 36916.871 | 55333.372 |
| Cmax | 1419.038 | 3052.336 |
| Tmax | 28.00 | 7.00 |

| | | |
|---|---|---|
| * AUC: pg·d/mL, Cmax: pg/mL, Tmax & t_{1/2}: d | | |

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

### Industrial Applicability

The present invention relates to a sustained-release injectable composition containing exenatide and a method for preparing the same.

## Claims

1. A sustained-release injectable composition containing exenatide, which comprises microparticles,
wherein the microparticles comprise exenatide and a biodegradable polymer, and
the area under the plasma level-time curve (AUC) for the release of exenatide for 63 days after administering the sustained-release injectable composition to SD rats at an exenatide dose of 8 mg/kg is 40,000 pg·d/mL to 100,000 pg·d/mL.

2. The sustained-release injectable composition of claim 1, wherein, as the sustained-release injectable composition is administered to SD rats at an exenatide dose of 8 mg/kg, it exhibits an effect of releasing exenatide rapidly at an initial stage, and then maintains a state in which the plasma concentration of exenatide is steady.

3. The sustained-release injectable composition of claim 1, wherein the sustained-release injectable composition releases exenatide in a sustained manner for 1 to 2 months.

4. The sustained-release injectable composition of claim 1, wherein the microparticles comprise exenatide and the biodegradable polymer at a weight ratio of 1:3 to 1:7.

5. The sustained-release injectable composition of claim 1, wherein the microparticles have an average diameter of 40 µm to 80 µm.

6. The sustained-release injectable composition of claim 1, wherein the biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

7. The sustained-release injectable composition of claim 1, wherein the injectable composition comprises a suspending solvent.

8. A method for preparing a sustained-release injectable composition containing exenatide, comprising:
step 1) of preparing an oil phase solution by mixing exenatide and a biodegradable polymer with an organic solvent;
step 2) of preparing a water phase solution by dissolving a surfactant in purified water;
step 3) of injecting the oil phase solution and the water phase solution into microchannels, respectively, and allowing the oil phase solution and the water phase solution to flow, thereby producing microparticles at an intersection formed between the microchannels;
step 4) of collecting the microparticles in a bath containing the water phase solution;
step 5) of removing the organic solvent from the collected microparticles;
step 6) of washing the microparticles, from which the organic solvent has been removed, with purified water, followed by drying; and
step 7) of mixing the dried microparticles with a suspending solvent, thereby preparing the sustained-release injectable composition,
wherein the area under the plasma level-time curve (AUC) for the release of exenatide for 63 days after administering the sustained-release injectable composition to SD rats at an exenatide dose of 8 mg/kg is 40,000 pg·d/mL to 100,000 pg·d/mL.

9. The method of claim 8, wherein the oil phase solution is prepared by dissolving exenatide in a co-solvent and then mixing the solution with the biodegradable polymer and the organic solvent.

10. The method of claim 8, wherein the co-solvent is selected from the group consisting of acetic acid, dimethyl sulfoxide, dimethylformamide, tetrahydrofuran, ethyl acetate, acetonitrile, and mixtures thereof.

11. The method of claim 9, wherein the co-solvent is contained at a weight ratio of 1:1 to 1:2 relative to the organic solvent.

12. The method of claim 8, wherein the oil phase solution is injected into the microchannel under a pressure condition of 500 mbar to 700 mbar, and then the pressure is increased at a rate of 1 to 5 mbar/min.

13. The method of claim 8, wherein the water phase solution is injected into the microchannel under a pressure condition of 2,500 mbar to 3,000 mbar.

14. The method of claim 8, wherein step 5) comprises stirring at a speed of 200 to 400 rpm for 4 to 6 hours at 40 to 50°C.
